# EUROPEAN PATENT APPLICATION

(11) **EP 4 159 836 A1**
(43) Date of publication of application: **05.04.2023**
(21) Application number: 21812210.9
(22) Date of filing: 16.02.2021
(51) Int. Cl.: C12M 1/26, C12Q 1/04

(54) **FILTRATION CARTRIDGE AND MICROBIAL TEST METHOD**

(30) Priority: 29.05.2020 JP 2020094439
(71) Applicant: HITACHI HIGH-TECH CORPORATION, Tokyo 105-6409 (JP)
(72) Inventor: NODA Hideyuki, Tokyo 100-8280 (JP); ISHIMARU Masako, Tokyo 100-8280 (JP)
(74) Representative: Mewburn Ellis LLP
(86) International application number: PCT/JP2021/005769
(87) International publication number: WO 2021/240911

(57) **Abstract**

Provided is a technique for suppressing false positives due to environmental contamination and easily performing a highly sensitive and highly accurate microbial test. A filtration cartridge according to the present disclosure includes a filter cup with a filter capturing microorganisms, a first filter cup attachment that covers a lower portion of the filter cup and has at least one through-hole in a bottom surface portion, and a second filter cup attachment, being connected to the first filter cup attachment, that covers an upper portion of the filter cup, and that has a lid installed at a position away from an opening surface of the filter cup.

## Description

### Technical Field

The present disclosure relates to a filtration cartridge and a microbial test method.

### Background Art

In the field of recent regenerative medicine products including cell therapeutic agents and gene therapeutic agents, the Act on Securing Quality, Efficacy, and Safety of Products Including Pharmaceuticals and Medical Devices (pharmaceuticals and medical device law) and the Act on Securing Safety of Regenerative Medicine are implemented. In the pharmaceuticals and medical device law, a ministry ordinance as a standard for production management and quality management of regenerative medicine products (GCTP ministry ordinance: Good Gene, Cellular, and Tissue-based Products Manufacturing Practice) is implemented, and a "sterile operation and like zone" and a "cleanliness control zone" are defined as environmental zones. In the ministry ordinance, the "sterile operation and the like zone" is defined as a location where a preparation operation of products and the like that need to be handled by a sterile operation is performed, a location where sterilized containers and the like are exposed to air in an operation place, and a location where the sterile operation such as a "sterility test" is performed in the operation place. Specifically, the locations defined in the ministry ordinance are classified into an important zone: grade A (in safety cabinet, in clean bench, in isolator, or the like), a direct support zone: grade B, and other support zones: grade C or D. In the sterility test, a sterile operator, a sterilized material, and the like are present in grade B, and a sterile dispensing operation of test samples and reagents is performed in grade A. Grade C or D is an area where a pre-sterilized material is handled as a zone less affected by product contamination.

Hitherto, a culturing method has been used in a microbial test and a sterility test for guaranteeing cleanliness of a production facility and sterility of water (pharmaceutical water) and a product itself used for production. In particular, a membrane filter method (MF method) is generally employed. The MF method is a method of filtering a collected sample through a membrane filter, then placing on a membrane filter obtained by filtering the sample on an agar medium, culturing the sample in a thermostat for up to 14 days, and visually counting the number of colonies of grown bacteria. However, as research on a wide variety of microorganisms has advanced, it has become clear that, since bacteria that grow relatively quickly until the colonies are visually detected require a period of at least several days and a culture medium of the related art has low colony-forming ability, there are bacteria that are difficult to be detected and quantified only by the culturing method. Rapid microbial test techniques using various measurement principles that do not necessarily require culture, which are different from culturing methods of the related art such as the MF method, have been expected as means for solving these problems.

The rapid microbial test methods are roughly classified into a direct detection method and an indirect detection method. The former includes solid phase cytometry, flow cytometry, and the like. The latter includes immunologic methods, nucleic acid amplification methods, bioluminescence, fluorescence (staining) methods, and the like. Although performance to be prioritized most in performance required for speeding up is sensitivity, improvement in sensitivity usually leads to a decrease in specificity. For example, Adenosine triphosphate (ATP) bioluminescence method (hereinafter, referred to as an ATP method) is a method of converting ATP present in a body cell into light and measuring the ATP by using a luminescence reaction of fireflies. In the ATP method, since presence of ATP molecules contained per cell can be detected as tens of thousands of photons, light corresponding to one cell is detected with high sensitivity by photon counting light measurement.

In the ATP method, in order to detect an extremely small amount (several bacteria level) of bacteria mixed in a specimen with high sensitivity and high accuracy, performance of a light emission measurement device must be highly sensitive as well as it is necessary to suppress a false light emission factor due to contamination such as ATP and bacteria present in an environment, microorganisms possessed by humans, and dust adsorbing ATP. In general, since ATP and free ATP derived from dead bacteria are mixed in the specimen, it is difficult to accurately estimate the number of viable bacteria when the specimen is used as it is for luminescence measurement.

PTL 1 describes that a specimen liquid is filtered and microorganisms and dead bacteria are decomposed in advance by using an ATP-degrading enzyme such as apyrase in order to remove the free ATP in the specimen.

### Citation List

### Patent Literature

PTL 1: WO 2016/147313A

### Summary of Invention

### Technical Problem

Attempts have been made to achieve both high sensitivity and high specificity not only in the ATP method but in all the rapid microbial test methods. A special pre-treatment step may be added to eliminate a substance as a false positive factor from contaminants included in a test target. Thus, the number of steps of complicated operations such as reaction with a plurality of reagents, separation and cleaning of a substance using filtration, centrifugation, and the like increases. Thus, there is a problem that contamination from the environment (floating, adhesion, and human-derived) occurs, false positives occur, and the specificity also decreases.

Therefore, when the rapid test method is applied, it is often necessary to bring a device such as a centrifuge or an incubator into an area having a level of grade A represented by the sterile operation zone such as the safety cabinet or the isolator. As a result, because a risk of environmental break of grade A increases due to bringing the device, a validity test (validation) is required for proving that the level of grade A is guaranteed. As a result, an operator requires a large amount of labor to introduce a new technique, and may give up introduction while understanding an advantage of the rapid test.

Thus, it is desired to realize a pre-treatment kit for the microbial test, a dedicated reaction container or a cartridge for pre-treatment, and automation based on a completely closed system, which are excellent in operability and capable of suppressing intrusion of contaminants from the environment.

Therefore, the present disclosure provides a technique for suppressing false positives due to environmental contamination and easily performing a highly sensitive and highly accurate microbial test.

### Solution to Problem

A filtration cartridge according to the present disclosure includes a filter cup with a filter capturing microorganisms, a first filter cup attachment that covers a lower portion of the filter cup and has at least one through-hole in a bottom surface portion, and a second filter cup attachment, being connected to the first filter cup attachment, that covers an upper portion of the filter cup, and that has a lid installed at a position away from an opening surface of the filter cup.

Further features related to the present disclosure will become apparent from the description of the present specification and the accompanying drawings. The aspects of the present disclosure are achieved and realized by elements, combinations of various elements, the following detailed description, and aspects of the appended claims.

The description of the present specification is merely a typical example, and does not limit the scope of claims or application examples of the present disclosure in any sense.

### Advantageous Effects of Invention

According to the filtration cartridge of the present disclosure, it is possible to suppress false positives due to environmental contamination and to easily perform a highly sensitive and highly accurate microbial test.

Other objects, configurations, and effects will be made apparent in the descriptions of the following embodiments.

### Brief Description of Drawings

[FIG. 1] FIG. 1 is a schematic cross-sectional view illustrating a filtration cartridge according to a first embodiment.
[FIG. 2A] FIG. 2A is a side view of a filter cup according to the first embodiment.
[FIG. 2B] FIG. 2B is a bottom view of the filter cup according to the first embodiment.
[FIG. 3A] FIG. 3A is a schematic cross-sectional view illustrating a configuration example of a first filter cup attachment according to the first embodiment.
[FIG. 3B] FIG. 3B is a schematic cross-sectional view illustrating another configuration example of the first filter cup attachment according to the first embodiment.
[FIG. 3C] FIG. 3C is a schematic cross-sectional view illustrating another configuration example of the first filter cup attachment according to the first embodiment.
[FIG. 3D] FIG. 3D is a schematic cross-sectional view illustrating another configuration example of the first filter cup attachment according to the first embodiment.
[FIG. 4A] FIG. 4A is a schematic front view of a second filter cup attachment according to the first embodiment.
[FIG. 4B] FIG. 4B is a schematic side view of the second filter cup attachment according to the first embodiment.
[FIG. 4C] FIG. 4C is a schematic front view illustrating another configuration example of the second filter cup attachment according to the first embodiment.
[FIG. 5] FIG. 5 is a schematic diagram illustrating a state where a suction filtration attachment and the filtration cartridge are connected.
[FIG. 6A] FIG. 6A is a schematic diagram illustrating a state where a first centrifugal filtration attachment and the filtration cartridge are connected.
[FIG. 6B] FIG. 6B is a schematic diagram illustrating a state where a second centrifugal filtration attachment and the filtration cartridge are connected.
[FIG. 6C] FIG. 6C is a schematic diagram illustrating a modification example in which the first centrifugal filtration attachment also serves as a body portion.
[FIG. 6D] FIG. 6D is a schematic diagram illustrating a modification example in which the second centrifugal filtration attachment also serves as a body portion.
[FIG. 7] FIG. 7 is a schematic diagram illustrating a biological clean room.
[FIG. 8] FIG. 8 is a flowchart illustrating a rapid microbial test method.
[FIG. 9] FIG. 9 is a diagram illustrating a photon counting time course using the filtration cartridge and the suction filtration attachment.
[FIG. 10] FIG. 10 is a flowchart illustrating a rapid microbial test method using the filtration cartridge and the centrifugal filtration attachment.
[FIG. 11] FIG. 11 is a flowchart illustrating a rapid microbial test method using the filtration cartridge, the suction filtration attachment, and the centrifugal filtration attachment.
[FIG. 12] FIG. 12 is a schematic diagram illustrating a microbial test apparatus according to a fifth embodiment.
[FIG. 13] FIG. 13 is a schematic diagram illustrating a microbial test apparatus according to a sixth embodiment.

### Description of Embodiments

Hereinafter, embodiments of the present disclosure will be described with reference to the accompanying drawings. However, each embodiment is merely an example for realizing the technique of the present disclosure, and does not limit the technology of the present disclosure. In the drawings, common components are denoted by the same reference signs.

### [First Embodiment]

### <Overall Configuration Example of Filtration Cartridge>

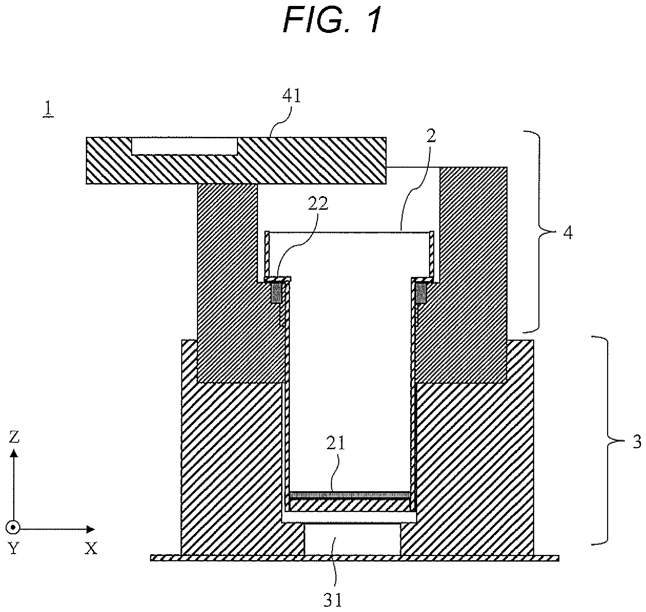

FIG. 1 is a schematic cross-sectional view illustrating a filtration cartridge 1 used for a microbial test according to a first embodiment. As illustrated in FIG. 1, the filtration cartridge 1 includes a filter cup 2, a first filter cup attachment 3, and a second filter cup attachment 4.

The filter cup 2 is provided, at its bottom portion, with a filter 21 having a pore diameter that basically does not allow passage of microorganisms such as bacteria, fungi, and archaea. The test sample is filtered to capture bacteria on the filter 21 by adding a liquid test sample to the filter cup 2 and causing the test sample to pass through the filter 21, and the test sample can be concentrated by removing filtrate.

A flange 22 is provided on an upper portion of the filter cup 2. An opening portion of the flange 22 serves as an inlet port for introducing the test sample and reagents into the filter cup 2.

The first filter cup attachment 3 covers a lower portion of the filter cup 2 and prevents contamination of an outer wall of the filter cup 2 and the filter 21. The first filter cup attachment 3 functions as a contamination protection cover for preventing an operator's finger or a surface such as a bench top, a ceiling, a side wall, or a glass window within an operation area from directly coming into contact with the filter cup 2.

A filtration nozzle 31 (through-hole) is provided at a bottom portion of the first filter cup attachment 3. As a result, a liquid sample such as the test sample or the reagent added to the filter cup 2 passes through the filter 21, and thus it becomes possible to forcibly pass the liquid sample from an upper portion to a lower portion of the filter 21.

The second filter cup attachment 4 is connected to the first filter cup attachment 3 and covers the upper portion of the filter cup 2, in particular the flange 22, and a lid 41 is provided at a position away from an opening surface of the filter cup 2 by a certain distance. As a result, it is possible to prevent dust from entering the filter cup 2 and to prevent the operator's hand or arm from touching the filter cup 2. Generally, an operator who uses the filtration cartridge 1 operates the filtration cartridge in a state where the lid 41 is closed in order to avoid a risk of entering of dust and contamination from the operator or the inside of the operation area, and temporarily opens the lid 41 for use in an operation of dispensing a sample to be tested and reagents for test into the filter cup 2. The lid 41 is configured to be movable parallel to the opening surface of the filter cup 2.

The filtration cartridge 1 can be provided to a user as a microbial test kit together with a suction filtration attachment and a centrifugal filtration attachment to be described later. The filtration cartridge 1 can be disposable.

### <Configuration Example of Filter Cup>

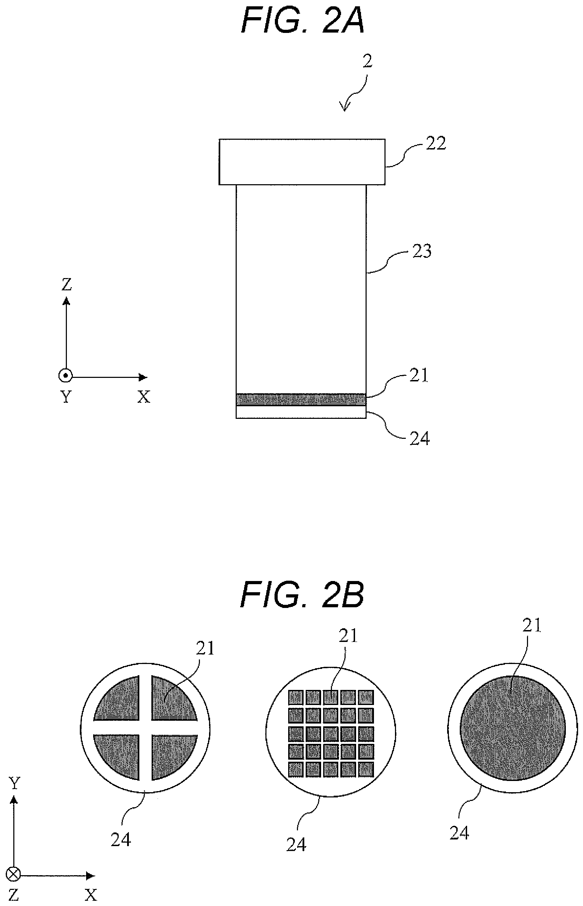

FIG. 2A is a side view of the filter cup 2. As illustrated in FIG. 2A, the filter cup 2 includes the filter 21, the flange 22, a container portion 23, and a support plate 24. The filter 21 is provided at a bottom portion of the container portion 23 and is supported by the support plate 24.

The container portion 23 and the support plate 24 can be made of, for example, a resin such as polypropylene, polystyrene, or polyethylene, and can be produced by injection molding, machining, or the like. The filter 21, the container portion 23, and the support plate 24 can be fixed by, for example, adhesion using ultrasonic welding. From the viewpoint of a fluid, a shape of the container portion 23 can be a cylindrical shape, but can also be a square tubular shape or a polygonal tubular shape, each having a corner depending on the application.

The container portion 23 is provided, on its upper portion, with the flange 22 for supporting the filter cup 2 by the second filter cup attachment 4. When the filter cup 2 is inserted into the second filter cup attachment 4, the flange 22 serves as a stopper, and thus the filter cup 2 is fixed at a predetermined position.

The filter 21 may be any filter capable of capturing a microbial to be tested, and for example, a membrane filter can be used. For example, cellulose mixed ester, PTFE, polyvinylidene fluoride (PVDF), polycarbonate, and the like can be used as a material of the filter 21.

A pore size of the filter 21 can be appropriately selected in accordance with a size of the microorganism to be tested and various conditions of the microbial test. The pore size can be equal to or greater than, for example, 0.20 um. Specifically, membrane filters having pore sizes of 0.22 um, 0.45 um, and the like can be enumerated as an example of the filter 21.

It is possible to minimize liquid remaining after filtration as much as possible by setting a size (diameter) of the filter 21 and a diameter of the container portion 23 to the same diameter and by straightening both an inner wall of the container portion 23 and a side surface of the filter 21. Alternatively, it is possible to minimize the liquid remaining after filtration as much as possible by setting to be the same a shape of the side surface of the filter 21 and a shape of the bottom portion of the container portion 23. Of course, the container portion 23 may be formed in a tapered shape that becomes thinner from the upper portion toward the bottom portion. Depending on the application, although the container portion 23 may have a tapered shape that becomes thicker from the upper portion toward the bottom portion, since a large distribution may occur in the amount of liquid per unit time passing through a filtration surface of the filter 21 during filtration by suction or centrifugation, clogging is caused. Although the container portion 23 may have a structure bent in the middle depending on the application, uniformity of the amount of liquid passing through the filtration surface may be lost, and a large distribution may occur. Thus, clogging is caused.

The diameter of the filter 21 can be, for example, several mm to 47 mm. A diameter of the bottom portion of the container portion 23 is substantially equal to the diameter of the filter 21. A height of the container portion 23 is designed in accordance with the amount of liquid of the test sample, and can be designed such that several µL to several 100 mL can be accommodated, for example.

However, a case where an area and a volume for capturing microorganisms are reduced by decreasing the diameter of the filter 21 as much as possible is useful for improving sensitivity since a sample concentration (per unit area or per unit liter) used for measurement is increased. When the area of the filter 21 is reduced, a large volume of test sample can be filtered by attaching a funnel to the opening portion of the filter cup 2.

FIG. 2B is a bottom view of the filter cup 2. As illustrated in the left diagram of FIG. 2B, the support plate 24 can be formed by a plurality of support rods radially extending from a center, represented by a cross shape. As illustrated in the center diagram of FIG. 2B, the support plate 24 can support the filter 21 by a mesh structure having a plurality of through-holes of levels from several mm to several cm. As illustrated in the right diagram of FIG. 2B, the support plate 24 may have a shape of supporting only an end portion of the filter 21. As described above, the support plate 24 has the through-hole capable of passing through the liquid having passed through the filter 21.

### <Configuration Example of First Filter Cup Attachment>

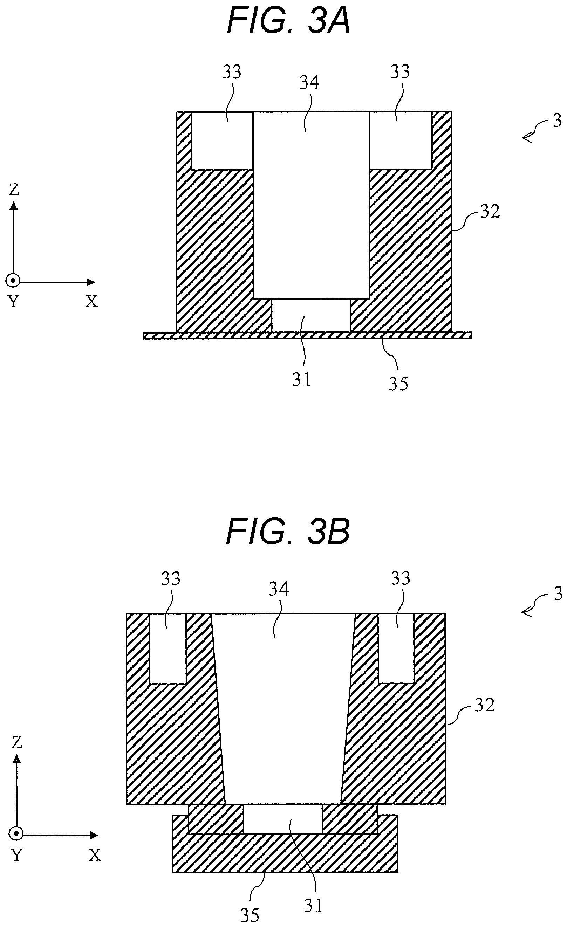

FIG. 3A is a schematic cross-sectional view illustrating a configuration example of the first filter cup attachment 3. As described above, the first filter cup attachment 3 prevents contamination of the outer wall of the filter cup 2 and the filter 21. The first filter cup attachment 3 can be made of, for example, a resin such as polypropylene, polystyrene, or polyethylene and can be produced by injection molding, machining, or the like.

As illustrated in FIG. 3A, the first filter cup attachment 3 has a body portion 32 in which the filtration nozzle 31, an attachment joint hole 33, and a filter cup insertion portion 34 are formed. A sealing material 35 is provided on a lower surface of the body portion 32.

The attachment joint hole 33 and the filter cup insertion portion 34 correspond to recesses fitted to the outer shape of the container portion 23 of the filter cup 2, and the filter cup 2 is inserted into these recesses. The filter cup insertion portion 34 is coupled to the filtration nozzle 31. The filter cup 2 and the first filter cup attachment 3 are fixed by, for example, press-bonding by pressing. A part of the body portion of the second filter cup attachment 4 to be described later is also included in the first filter cup attachment 3.

Since the operator may remove the filter cup 2 from the first filter cup attachment 3 during a step of the microbial test in which the filtration cartridge 1 is used, an inner diameter of the first filter cup attachment 3 is formed to be larger than an outer diameter of the container portion 23 of the filter cup 2.

A connector (claw-like connector or tubular connector to be described later) of the second filter cup attachment 4 is fitted into the attachment joint hole 33, and thus, the first filter cup attachment 3 is held in state of being close contact with the filter cup 2. As a result, the filter cup 2 is more stably stored in the first filter cup attachment 3 and the second filter cup attachment 4. The attachment joint hole 33 illustrated in FIG. 3A is a form suitable for press-bonding by pressing or the second filter cup attachment 4 including a tubular connector. When the tubular connector is a male screw, a wall surface of the attachment joint hole 33 can have a female tap shape.

The first filter cup attachment 3 is used in connection with a suction filtration attachment, a first centrifugal filtration attachment, and a second centrifugal filtration attachment to be described later. Thus, the bottom portion of the body portion 32 is inserted into recesses provided in the suction filtration attachment, the first centrifugal filtration attachment, and the second centrifugal filtration attachment.

An inner diameter of the filtration nozzle 31 can be set to be equal to or greater than at least 0.5 mm and can be set to be increased as much as possible from the viewpoint of allowing a substance having passed through the filter 21 to pass therethrough without capturing the substance and from the viewpoint of efficiently discharging the liquid. By setting the inner diameter of the filtration nozzle 31 to, for example, 0.5 mm to 10 mm, provided is the filtration nozzle 31 which is excellent in compatibility with an inner diameter of a general-purpose tube used for suction.

An opened portion of the filtration nozzle 31 is provided to the operator in a state of being sealed with the sealing material 35 until immediately before use by the operator. Thus, an inside of the first filter cup attachment 3 is prevented from coming into contact with outside air or coming into contact with the operator or a surface of an object. The sealing material 35 can be removed by the operator. The sealing material 35 can be, for example, a tape made of metal or resin.

FIG. 3B is a schematic cross-sectional view illustrating another configuration example of the first filter cup attachment 3. In the example illustrated in FIG. 3B, the attachment joint hole 33 has a structure suitable for the second filter cup attachment 4 including the claw-like connector.

In the example illustrated in FIG. 3B, the filter cup insertion portion 34 has a slightly tapered structure in which an inner diameter decreases along a direction of the filtration nozzle 31. As a result, the filter cup 2 can be more easily removed from the first filter cup attachment 3.

In the example illustrated in FIG. 3B, the outer diameter of a formed portion of the filtration nozzle 31 is smaller than the outer diameter of the body portion 32. The opened portion of the filtration nozzle 31 is sealed with the sealing material 35 in the form of a cap (for example, made of resin).

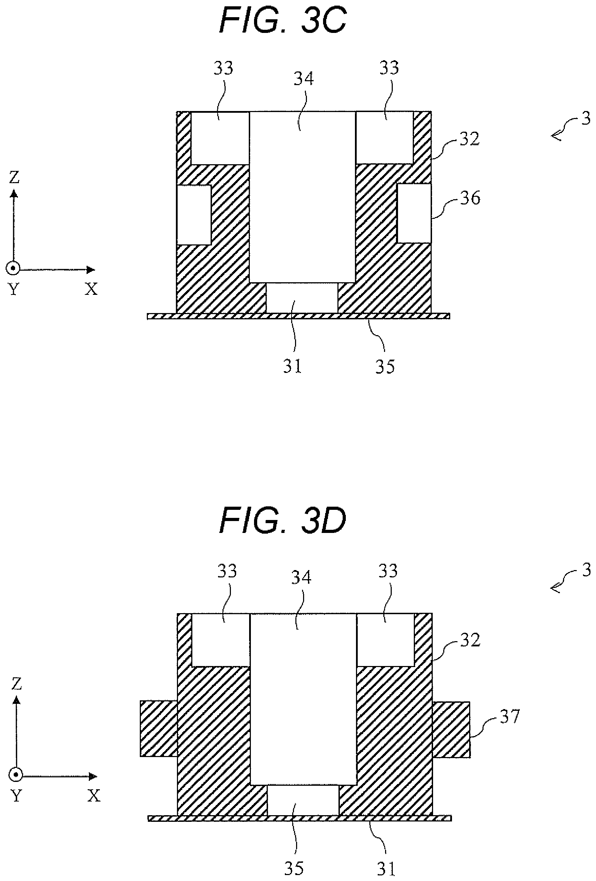

FIGS. 3C and 3D are schematic cross-sectional views illustrating another configuration example of the first filter cup attachment 3. In the example illustrated in FIG. 3C, a recessed portion 36 is provided on an outer peripheral surface of the body portion 32, and in the example illustrated in FIG. 3D, a protrusion portion 37 is provided on the outer peripheral surface of the body portion 32. As a result, improved is ease of gripping the body portion by the operator, for example, in attaching and detaching the first filter cup attachment 3 to and from the suction filtration attachment, the first centrifugal filtration attachment, or the second centrifugal filtration attachment.

### <Configuration Example of Second Filter Cup Attachment>

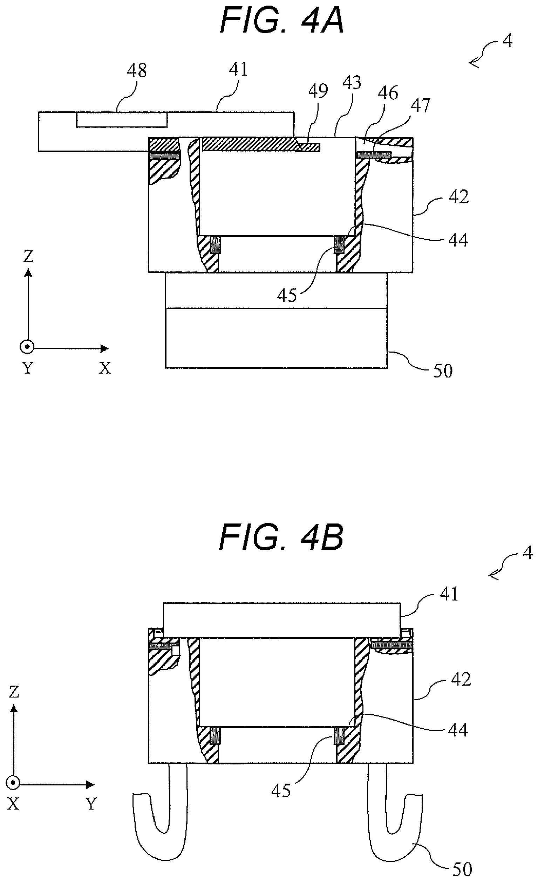

FIG. 4A is a schematic front view of the second filter cup attachment 4. FIG. 4B is a schematic side view of the second filter cup attachment 4. In FIGS. 4A and 4B, a cross section of the second filter cup attachment 4 is partially illustrated.

As illustrated in FIG. 4A, the second filter cup attachment 4 includes the lid 41, a body portion 42, and a claw-like connector 50. The body portion 42 is provided with a filter cup introduction hole 43, a flange holding portion 44, a first fixing member 45, a lid fixing hole 46, and a second fixing member 47.

A step as the flange holding portion 44 is provided in the filter cup introduction hole 43. An inner diameter of the filter cup introduction hole 43 at a lower stage is smaller than an inner diameter at an upper stage than the flange holding portion 44. The flange 22 of the filter cup 2 inserted from the filter cup introduction hole 43 abuts on the flange holding portion 44 to serve as a stopper. Thus, the filter cup 2 is held at a predetermined position. When the inner diameter of the filter cup introduction hole 43 and the outer diameter of the container portion 23 of the filter cup 2 are substantially the same, these components are press-bonded and fixed. The first fixing member 45 is inserted into a step portion of the filter cup introduction hole 43, and an outer periphery of the container portion 23 under the flange 22 is brought into close contact with the filter cup introduction hole. As a result, the fixing performance of the filter cup 2 is improved. The first fixing member 45 is, for example, an O-ring, a sealing tape, or the like.

When the lid 41 is closed, a projection portion 49 of the lid 41 enters into the lid fixing hole 46, and the lid is temporarily closed at a location where a taper angle of the lid fixing hole 46 changes. In this position, there is a minute gap into which the outside air flows, and thus, the lid is in a semi-closed state (first closed state). When the lid 41 is further pushed into the lid fixing hole 46, a sealed state for blocking the outside air is formed. This state is set to a fully closed state (second closed state). A position of the lid 41 in the semi-closed state is referred to as a "semi-closed position." The semi-closed position is a position required to create a necessary air flow during suction filtration. On the other hand, a position of the lid 41 in the fully closed state is referred to as a "fully closed position". The fully closed position is a position necessary for preventing contamination of airborne microorganisms when being taken out from a sterile operation zone to a peripheral zone. The second fixing member 47 is, for example, an O-ring, a sealing tape, or the like, and prevents unintended opening of the lid 41. As described above, the lid 41 can take the two-stage closed state.

A knob 48 is a recess provided in an upper surface of the lid 41. The operator hooks his or her finger on the knob 48 during an opening and closing operation of the lid 41. As illustrated in FIG. 4A, the knob 48 has a recessed shape, but may have a protruding shape.

The claw-like connector 50 is provided to project from a bottom surface of the body portion 42. As illustrated in FIG. 4B, the claw-like connector 50 has a substantially U-shape curved outward. When the second filter cup attachment 4 is connected to the above-described first filter cup attachment 3, the claw-like connector 50 is inserted into the attachment joint hole 33. At this time, a curved portion of the claw-like connector 50 is distorted, and joined to the attachment joint hole 33 illustrated in FIG. 3B by using a relationship between a key and a key hole.

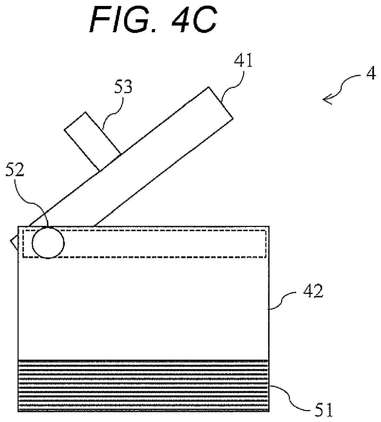

FIG. 4C is a schematic front view illustrating another configuration example of the second filter cup attachment 4. In the example illustrated in FIG. 4C, the lid 41 is in the form of a hinge mechanism 52 that opens and closes at an angle of, for example, 90° or more and less than 180° with respect to the opening surface of the filter cup 2. Provided is the lid 41 which is excellent in the operability of opening and closing also by such a form. A knob 53 corresponding to a protrusion portion is provided in the lid 41 of the hinge mechanism 52. In the example illustrated in FIG. 4C, a tubular connector 51 is provided on the bottom surface of the body portion 42. The tubular connector 51 is a tubular male screw, and is screwed to the attachment joint hole 33 (female tap shape) illustrated in FIG. 3A.

### <Conclusion of First Embodiment>

As described above, the filtration cartridge 1 according to the first embodiment includes the filter cup 2 having the filter 21 that captures bacteria at the bottom portion, the first filter cup attachment 3 that covers the lower portion and the bottom surface of the filter cup 2 and has at least one filtration nozzle 31 (through-hole) at the bottom portion, and the second filter cup attachment 4 that covers the upper portion of the filter cup 2 and has the lid 41 installed with a space at a position away from the uppermost surface of the filter cup 2. With such a configuration, the lid 41 can be closed at the time of use or transportation by the operator. Since the lid 41 does not come into contact with the opening portion of the filter cup 2, contamination of the filter cup 2 from the outside can be prevented. Since the operator may open the lid 41 when adding the test sample or the reagent to the filter cup 2, the operation is also simple.

At the time of producing the filtration cartridge 1, it is possible to assemble the filter cup 2, the first filter cup attachment 3, and the second filter cup attachment 4 under an environment of grade A of the sterile operation zone described above. Since the lid 41 is sealed and the filtration nozzle 31 is sealed with the sealing material 35 at the time of being provided to the operator, it is possible to maintain a level of grade A for the filter cup 2 disposed inside even when the filtration cartridge 1 is transported from a zone of grade B or the like to a zone of grade A. As a result, it is not necessary to bring a device such as a centrifuge or an incubator into grade A. In addition, the sterile operation is facilitated without increasing a risk of environmental break due to deterioration of a complete sterile environment in grade A due to bringing the device.

As described above, according to the filtration cartridge 1 of the first embodiment, it is possible to suppress false positive due to environmental contamination, and it is possible to easily perform a highly sensitive and highly accurate microbial test (particularly, MF method).

### [Second Embodiment]

In a second embodiment, the suction filtration attachment connected to the filtration cartridge will be described.

### <Configuration Example of Suction Filtration Attachment>

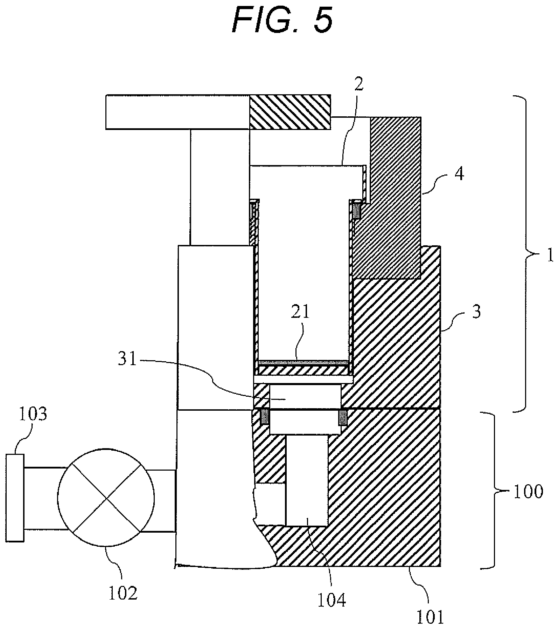

FIG. 5 is a schematic diagram illustrating a state where a suction filtration attachment 100 and the filtration cartridge 1 are connected. In FIG. 5, a cross section is partially illustrated. As illustrated in FIG. 5, the suction filtration attachment 100 includes a support block 101, an opening and closing valve 102, and a pipe connector 103 (connection structure portion). The suction filtration attachment 100 can be made of, for example, a resin such as polypropylene, polystyrene, or polyethylene.

The first filter cup attachment 3 is fitted to the support block 101, and thus the first filter cup attachment is supported. A flow path 104 is provided inside the support block 101. One end portion of the flow path 104 is connected to the filtration nozzle 31 of the first filter cup attachment 3. The other end portion of the flow path 104 is connected to a suction device (not illustrated) via the opening and closing valve 102 and the pipe connector 103. In other words, the flow path 104 connects the filtration nozzle 31 and the pipe connector 103. As a result, the liquid sample added to the filter cup 2 passes through the filter 21 of the filtration cartridge 1 and is filtered. The liquid (filtrate) other than microorganisms captured by the filter 21 passes through the flow path 104 of the suction filtration attachment 100 and then is discharged from the pipe connector 103.

### [Third Embodiment]

In a third embodiment, the centrifugal filtration attachment connected to the filtration cartridge will be described.

### <Configuration Example of Centrifugal Filtration Attachment>

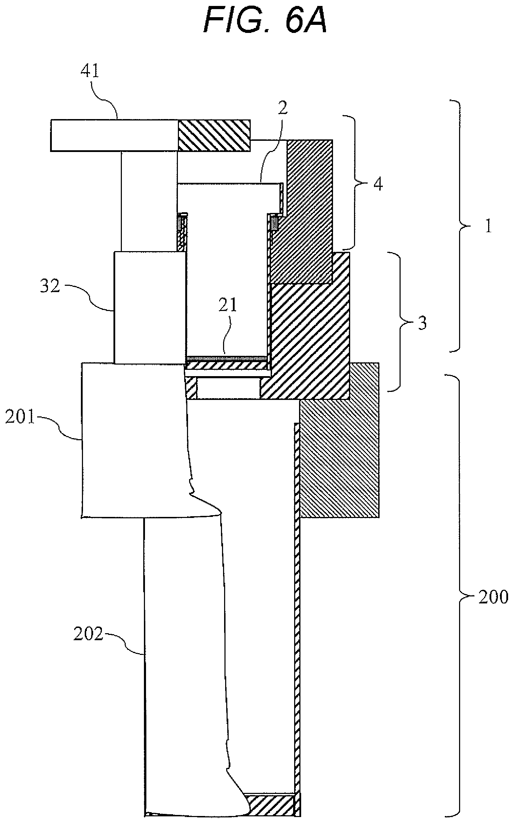

FIG. 6A is a schematic diagram illustrating a state where a first centrifugal filtration attachment 200 and the filtration cartridge 1 are connected. In FIG. 6A, a cross section is partially illustrated. As illustrated in FIG. 6A, the first centrifugal filtration attachment 200 includes a first fitting portion 201 and a waste liquid tube 202 (container). The first centrifugal filtration attachment 200 can be made of, for example, a resin such as polypropylene, polystyrene, or polyethylene. The body portion 32 of the first filter cup attachment 3 is fitted to the first fitting portion 201. The waste liquid tube 202 is inserted into the first fitting portion 201 on a side opposite to a side where the body portion 32 is fitted.

By setting in a centrifuge the first centrifugal filtration attachment 200 and the filtration cartridge 1 in a state of being connected to each other and then performing centrifugal separation, the liquid sample added to the filter cup 2 passes through the filter 21 and is filtered. A filtrate is collected in the waste liquid tube 202.

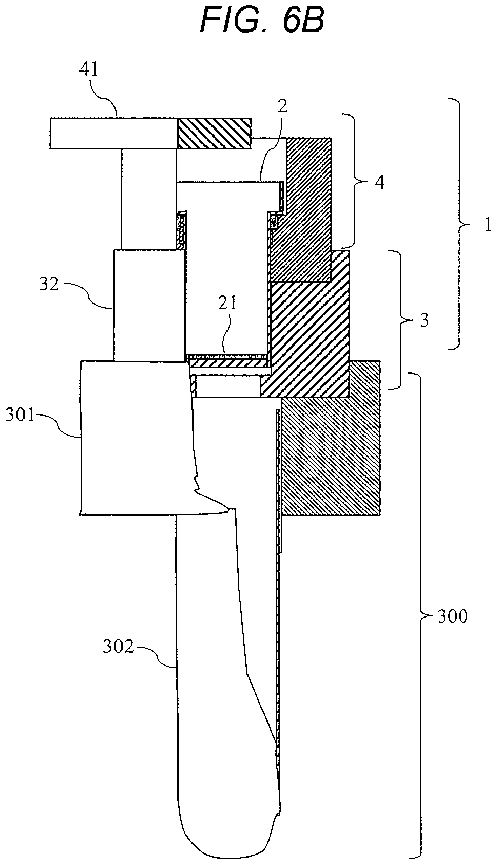

FIG. 6B is a schematic diagram illustrating a state where a second centrifugal filtration attachment 300 and the filtration cartridge 1 are connected. In FIG. 6B, a cross section is partially illustrated. As illustrated in FIG. 6B, the second centrifugal filtration attachment 300 includes a second fitting portion 301 and a measurement tube 302 (container). The body portion 32 of the filtration cartridge 1 is fitted to the second fitting portion 301. The measurement tube 302 is inserted into the second fitting portion 301 on a side opposite to a side where the body portion 32 is fitted.

By setting in a centrifuge the second centrifugal filtration attachment 300 and the filtration cartridge 1 in a state of being connected to each other and then performing centrifugal separation, the liquid sample added to the filter cup 2 passes through the filter 21 and is filtered. A filtrate is collected in the measurement tube 302.

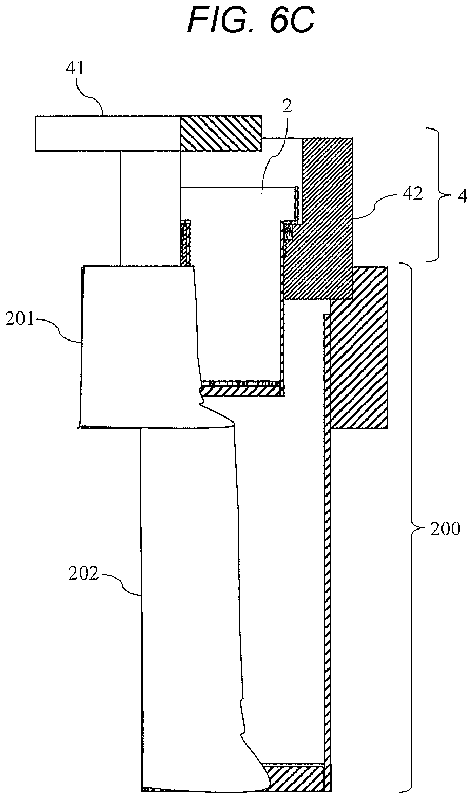

FIG. 6C is a schematic diagram illustrating a modification example in which the first centrifugal filtration attachment 200 also serves as the body portion 32. As illustrated in FIG. 6C, the body portion 42 of the second filter cup attachment 4 is connected to the first fitting portion 201 of the first centrifugal filtration attachment 200. In this case, the first fitting portion 201 is provided with an attachment joint hole for connecting the claw-like connector or the tubular connector of the body portion 42. The state illustrated in FIG. 6C is realized, for example, by the operator removing the first filter cup attachment 3 of the filtration cartridge 1 and then connecting the first centrifugal filtration attachment 200.

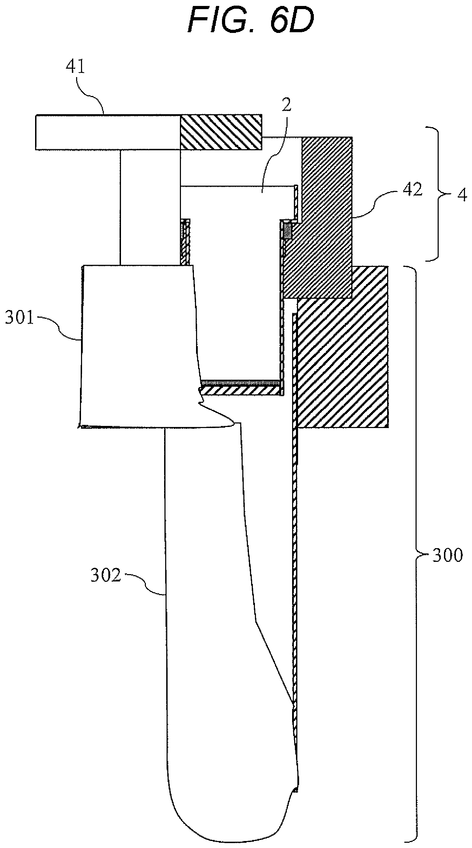

FIG. 6D is a schematic diagram illustrating a modification example in which the second centrifugal filtration attachment 300 also serves as the body portion 32. As illustrated in FIG. 6D, the body portion 42 of the second filter cup attachment 4 is connected to the second fitting portion 301 of the second centrifugal filtration attachment 300. The other points are similar to those in FIG. 6C.

### [Fourth Embodiment]

In a fourth embodiment, a microbial test method using the above-described filtration cartridge will be described. Here, it is supposed that the filtration cartridge of the present disclosure is applied to pharmaceutical water management. A microbial management standard of pharmaceutical water is managed as treatment standard values with an allowable amount of microorganisms contained in water, based on the pharmacopoeia of each country. In the Japanese Pharmacopoeia, maintenance of a pharmaceutical water production line is required when normal water exceeds 100 CFU/mL, purified water exceeds 10 CFU/mL, and injection water exceeds 10 CFU/100 mL. As the treatment standard values in the United States Pharmacopeia, 500 CFU/mL is defined for drinking water, 100 CFU/mL is defined for purified water, and 10 CFU/100 mL is defined for injection water. As the treatment standard values in the European Pharmacopeia, 100 CFU/mL is defined for purified water, 10 CFU/100 mL is defined for highly purified water, and 10 CFU/100 mL is defined for injection water.

Thus, in the following description, three kinds of normal water, purified water, and injection water are used as test samples, in order to perform a microbial test of pharmaceutical water conformity to the test of the Japanese Pharmacopoeia. 1 mL of normal water and purified water is used as a test volume, and only 100 mL of injection water is used.

In the microbial test of pharmaceutical water, it is possible to apply various microbial rapid test methods such as a fluorescence staining method. However, it is supposed, in the present embodiment, to use a rapid test method by an ATP luminescence method using a high-sensitivity ATP test kit (Lumione (registered trademark) rapid microbial test reagent kit (manufactured by Hitachi High-Tech Solutions Corporation)) and a high-sensitive ATP measurement device (Lumione BL-2000 (manufactured by Hitachi High-Tech Solutions Corporation)). The high-sensitivity ATP test kit includes an active erasure liquid, a cleaning liquid, a luminescent liquid, an extraction liquid, a positive control (ATP 100 amol/50 µL extraction liquid), a measurement tube, and a dedicated rack thereof (microbial rapid test apparatus that ensures quality of pharmaceutical products Lumione BL-2000, 2018, vol. 100, No. 6).

### <Microbial Test Environment>

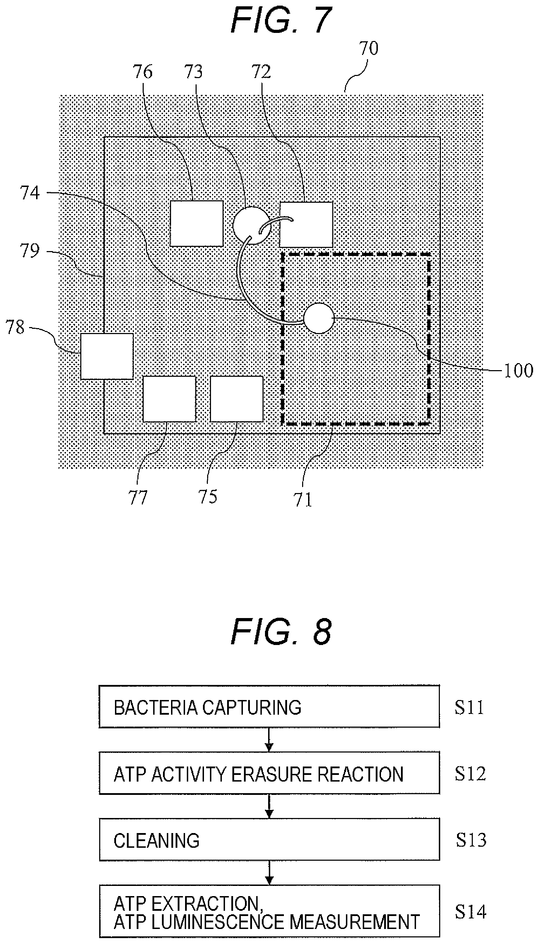

FIG. 7 is a schematic diagram illustrating a biological clean room 70. It is possible to perform the rapid test method by the ATP luminescence method in the biological clean room 70. As illustrated in FIG. 7, the biological clean room 70 is provided with a safety cabinet 71 of grade A as an important zone and a sterile operation zone 79 of grade B. The sterile operation zone 79 is installed with a suction pump 72 (suction device) and peripheral tools (waste liquid trap 73 and suction pump tube 74), an incubator 75, a centrifuge 76, and a measurement device 77. The operator in the sterile operation zone 79 can arbitrarily access the safety cabinet 71, the suction pump 72, and the peripheral tools (waste liquid trap 73 and suction pump tube 74), the incubator 75, the centrifuge 76, and the measurement device 77.

Here, grade A means a local sterile operation zone where an operation that needs to prevent a risk of contamination of products at a high level is performed. On the other hand, grade B means a multipurpose zone where an operation that needs to prevent the risk of contamination of products at a relatively high level is performed. That is, this zone of grade B is a zone where there are a person, an instrument, a device, and the like that carry in a container, a raw material, and an intermediate product after sterilization stored to be able to maintain sterility and that directly get involved in the sterile operation zone. In a typical sterile room, the zone of grade B is a surrounding environment of grade A.

The arrangement of FIG. 7 is merely an example. An actual site is installed with infrastructure facilities other than the facilities illustrated in FIG. 7 to maintain cleanliness. Cleaning and environmental monitoring in the biological clean room 70 are appropriately performed. For example, airborne bacteria are managed at less than 1 CFU/m³ in the safety cabinet 71 corresponding to grade A. The zone of grade B is a test zone in which airborne bacteria are managed at 10 CFU/m³ or less.

The operator can bring the test sample, the filtration cartridge 1, the suction filtration attachment 100, and at least one of the first centrifugal filtration attachment 200 and the second centrifugal filtration attachment 300, the test reagents, and a sample and reagent dispensing tool (for example, micropipette or Pipetman) to the sterile operation zone 79 via a pass box 78, and can introduce these devices in a state of being sterile-packed in the safety cabinet 71. The filtration cartridge 1 can be at least doubly packaged to remove one packaging before introduction into the safety cabinet 71 and the remaining packaging after introduction into the safety cabinet 71. The filtration cartridge 1 is packaged through sterilization steps such as gamma ray sterilization, EOG sterilization, and autoclaving.

The test reagents are the activity erasure liquid (hereinafter, referred to as a reagent A), the cleaning liquid (hereinafter, referred to as a reagent B), and the extraction liquid (hereinafter, referred to as a reagent C) included in the high-sensitivity ATP test kit. The luminescent liquid is set at a predetermined position of the measurement device 77.

<Rapid Microbial Test using Filtration Cartridge and Suction Filtration Attachment>

FIG. 8 is a flowchart illustrating the rapid microbial test method using the filtration cartridge 1 and the suction filtration attachment 100 (FIG. 5).

### (Step S11: Bacteria Capturing)

In step 511, the operator captures bacteria on the filter 21 of the filtration cartridge 1. Details of this step S11 are as follows.

First, the operator introduces the test sample, the filtration cartridge 1, the suction filtration attachment 100, the test reagents, and the sample and reagent dispensing tool into the safety cabinet 71, opens the filtration cartridge 1 and the suction filtration attachment 100, removes the sealing material 35 of the filtration nozzle 31, and connects the filtration cartridge 1 and the suction filtration attachment 100. One filtration cartridge 1 and one suction filtration attachment 100 are used for each of the test samples (1 mL of normal water, 1 mL of purified water, and 100 mL of injection water).

Next, as preparation for a filtration step of the test sample, the operator pulls out only the pipe connector 103 attached to the suction filtration attachment 100 from a front shutter of the safety cabinet 71 to the zone of grade B, connects the pipe connector to the suction pump tube 74 in the zone of grade B, and drives the suction pump 72.

Next, the operator opens the lid 41 of the second filter cup attachment 4 in the safety cabinet 71 to inject the test sample into the filter cup 2 from a container containing the test sample by a pipetting operation. After injecting a required amount of test sample into the filter cup 2, the operator closes the lid 41 of the second filter cup attachment 4 to the semi-closed position, and opens the opening and closing valve 102 of the suction filtration attachment 100 to start the filtration of the test sample. After starting the filtration, the operator visually observes the transparent or translucent suction pump tube 74. The operator opens the lid 41 when the flow of the liquid cannot be visually observed, and closes the lid 41 to the fully closed position after confirming that the liquid does not remain on the filter 21. Since there is no residual liquid, the operator determines that the filtration is completed, closes the opening and closing valve 102 of the suction filtration attachment 100, disconnects the suction filtration attachment 100 and the suction pump 72, to stop the filtration.

With the operations so far, liquid components of the test sample (1 mL of normal water, 1 mL of purified water, and 100 mL of injection water) pass through the filter 2. When there are bacteria in the filter 21, the liquid components are concentrated and the bacteria is captured by the filter 21.

### (Step S12: ATP Activity Erasure Reaction)

In step S12, the operator performs an ATP activity erasure reaction by using the reagent A of the Lumione test kit. Details of this step S12 are as follows.

The operator opens the lid 41 of the second filter cup attachment 4 to add a predetermined addition amount of reagent A by a pipetting operation, and then closes the lid 41 of the second filter cup attachment 4 to the fully closed position. In order to take out from the safety cabinet 71 the filtration cartridge 1 to which the suction filtration attachment 100 is connected, the operator removes the opening and closing valve 102 and the pipe connector 103 from the suction filtration attachment 100, and holds the opening and closing valve 102 and the pipe connector 103 in the safety cabinet 71. The operator introduces into the incubator 75 (zone of grade B) the filtration cartridge 1 to which the suction filtration attachment 100 is connected, to incubate the filtration cartridge 1 at 37°C for 40 minutes. After 40 minutes, the operator takes out from the incubator 75 the filtration cartridge 1 to which the suction filtration attachment 100 is connected, to perform alcohol cleaning. After the alcohol cleaning, the operator re-introduces the filtration cartridge into the safety cabinet 71, and then connects the opening and closing valve 102 and the pipe connector 103. After opening the lid 41 of the second filter cup attachment 4 to the semi-closed position, the operator opens the opening and closing valve 102 of the suction filtration attachment 100 to filter the reagent A. The operator visually observes the suction pump tube 74, opens the lid 41 when the flow of the liquid cannot be visually observed, and confirms that the liquid does not remain on the filter 21. Thereafter, the operator closes the opening and closing valve 102 of the suction filtration attachment 100 to stop filtration.

### (Step S13: Cleaning)

In step S13, the operator cleans the filter 21 by using the reagent B of the Lumione test kit. Details of this step S13 are as follows.

The operator opens the lid 41 of the second filter cup attachment 4 to add a predetermined addition amount of reagent B by a pipetting operation. The operator brings the lid 41 of the second filter cup attachment 4 to the semi-closed position and opens the opening and closing valve 102 of the suction filtration attachment 100 to filter the reagent B. The operator visually observes the suction pump tube 74 and opens the lid 41 when the flow of the liquid cannot be visually observed. The operator confirms that the liquid does not remain on the filter 21 and closes the opening and closing valve 102 to stop the filtration.

### (Step S14: ATP Extraction)

In step S14, the operator extracts ATP by using the reagent C of the Lumione test kit to complete the preparation of an ATP measurement sample. Details of this step S14 are as follows.

The operator opens the lid 41 of the second filter cup attachment 4 to add the reagent C in an addition amount of 50 µL defined in the manual of the kit by a pipetting operation. After adding the reagent C, the operator brings the lid 41 of the second filter cup attachment 4 to the fully closed position, and then holds the reagent C at a room temperature for about several to 10 minutes in a state of being immersed in the filter 21. Thereafter, the operator opens the lid 41 to collect the entire amount (50 µL) of reaction liquid (hereinafter, referred to as a "reagent C reaction liquid") after the reaction between the reagent C and the bacteria from the filter 21 by a pipetting operation. The operator dispenses the collected reaction liquid into the measurement tube. The operator sets the measurement tube in a dedicated tube rack and covers the tube rack.

Finally, the operator introduces the tube rack into the measurement device 77 installed in grade B to perform ATP measurement.

### (ATP Luminescence Measurement)

The high-sensitive ATP measurement device (Lumione BL-2000 (manufactured by manufactured by Hitachi High-Tech Solutions Corporation)) is used as the measurement device 77. When the operator sets the luminescent liquid of the high-sensitivity ATP test kit (Lumione rapid microbial test reagent kit (manufactured by Hitachi High-Tech Solutions Corporation)) in the measurement device 77 and sets a dedicated tube rack, to start measurement, the measurement device 77 sequentially measures the reagent C reaction liquid contained in up to 24 measurement tubes.

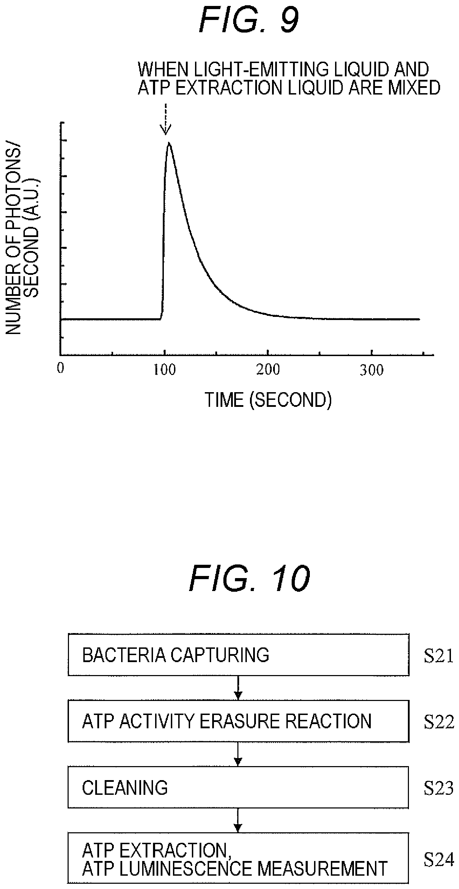

FIG. 9 is a graph of a typical photon counting time course obtained by automatically dispensing the luminescent liquid into the measurement tube into which the reagent C reaction liquid is introduced. An ATP amount is calculated by integrating light emission amounts of the photon counting time course for a certain time and normalizing an average value of the light emission amounts as a relative light unit (RLU).

The operator prepares at least three blank samples obtained by directly dispensing 50 µL of reagent C into the measurement tube in advance. The operator also prepares at least one standard positive sample obtained by dispensing a positive control (ATP 100 amol/50 µL extraction liquid) included in the test kit into the measurement tube. The operator quantifies an ATP amount of an actual test sample by measuring these samples before measurement of the reagent C reaction solution as the actual test sample and by creating a two-point calibration curve of 0 amol and 100 amol. This flow of the ATP measurement is standard performed in accordance with a manual performed by the measurement device of the Lumione BL-2000.

When an ATP amount corresponding to one viable bacteria is set to 1 amol or more and measurement is performed by using this amount as a threshold value, it can be determined that there are microorganisms in the sample when the ATP amount exceeding 1 amol is detected.

### <Rapid Microbial Test using Filtration Cartridge and Centrifugal Filtration Attachment>

FIG. 10 is a flowchart illustrating the rapid microbial test method using the filtration cartridge 1 and the first centrifugal filtration attachment 200 (FIG. 6A) and the second centrifugal filtration attachment 300 (FIG. 6B) .

### (Step S21: Bacteria Capturing)

In step S21, the operator captures bacteria on the filter 21 of the filtration cartridge 1. Details of this step S21 are as follows.

First, the operator introduces the test sample, the filtration cartridge 1, the first centrifugal filtration attachment 200, the second centrifugal filtration attachment 300, the test reagents, and the sample and reagent dispensing tool into the safety cabinet 71, opens the microbial test filtration cartridge 1 and the first centrifugal filtration attachment 200. Then, the operator removes the sealing material 35 of the filtration nozzle 31, and connects the filtration cartridge 1 and the first centrifugal filtration attachment 200.

Next, the operator opens the lid 41 of the second filter cup attachment 4 in the safety cabinet 71 to inject the test sample into the filter cup 2 from a container containing the test sample by a pipetting operation.

After injecting a required amount of test sample into the filter cup 2, the operator closes the lid 41 of the second filter cup attachment 4 to the fully closed position. Then, the operator takes out from the safety cabinet 71 the filtration cartridge 1 to which the first centrifugal filtration attachment 200 is connected. Further, the operator sets the filtration cartridge in the centrifuge 76 (zone of grade B) to perform centrifugal filtration. A centrifugal force (×g) can be equal to or less than 2000 ×g not to damage bacteria. Since a time required for centrifugation depends on the volume and viscosity of the test sample, it is necessary to grasp a time during which the whole amount is filtered, in advance for each test sample. The condition in the present embodiment is 1000 ×g for 1 minute.

After the centrifugation is stopped, the operator takes out from the centrifuge 76 the filtration cartridge 1 to which the first centrifugal filtration attachment 200 is connected. Then, after spraying the filtration cartridge 1 to which the first centrifugal filtration attachment 200 is connected with alcohol to perform cleaning, the operator introduces the filtration cartridge into the safety cabinet 71. Next, the operator opens the lid 41 of the second filter cup attachment 4 and confirms that there is no residual liquid. Here, when the liquid remains, the centrifugal filtration by the centrifuge 76 may be performed again.

With the operations so far, liquid components of the test sample (1 mL of normal water, 1 mL of purified water, and 100 mL of injection water) pass through the filter 21. When there are bacteria in the filter 21, the liquid components are concentrated and captured by the filter 21.

### (Step S22: ATP Activity Erasure Reaction)

In step S22, the operator performs the ATP activity erasure reaction by using the reagent A of the Lumione test kit. Details of this step S22 are as follows.

The operator opens the lid 41 of the second filter cup attachment 4 to add a predetermined addition amount of reagent A by a pipetting operation. Then, the operator closes the lid 41 of the second filter cup attachment 4 to the fully closed position. Next, the operator introduces into the incubator 75 (zone of grade B) the filtration cartridge 1 to which the first centrifugal filtration attachment 200 is connected, to incubate the filtration cartridge 1 at 37°C for 40 minutes. After 40 minutes, the operator takes out from the incubator 75the filtration cartridge 1 to which the first centrifugal filtration attachment 200 is connected. Then, the operator sets the filtration cartridge in the centrifuge 76 (zone of grade B) to perform centrifugal filtration. A centrifugal force (×g) can be equal to or less than 2000 ×g not to damage bacteria. Since a time required for centrifugation depends on a degree of clogging of the filter 21 by components of the test sample, it is necessary to grasp a time for filtering the entire amount of reagent A, in advance for each test target. The condition in the present embodiment is 1000 ×g for 1 minute.

After the centrifugation is stopped, the operator takes out from the centrifuge 76the filtration cartridge 1 to which the first centrifugal filtration attachment 200 is connected. After spraying the filtration cartridge with alcohol to clean the filtration cartridge, the operator introduces the filtration cartridge into the safety cabinet 71. Next, the operator opens the lid 41 of the second filter cup attachment 4 and confirms that there is no residual liquid. Here, when the liquid remains, the centrifugal filtration by the centrifuge 76 may be performed again after the lid 41 is closed to the fully closed position.

### (Step S23: Cleaning)

In step S23, the operator cleans the filter 21 by using the reagent B of the Lumione test kit. Details of this step S23 are as follows.

The operator opens the lid 41 of the second filter cup attachment 4 to add a predetermined addition amount of reagent B by a pipetting operation. Then, the operator closes the lid 41 of the second filter cup attachment 4 to the fully closed position. The operator takes out from the safety cabinet 71 the filtration cartridge 1 to which the first centrifugal filtration attachment 200 is connected. The operator sets the filtration cartridge in the centrifuge 76 (zone of grade B) to perform centrifugal filtration. A centrifugal force (×g) can be equal to or less than 2000 ×g not to damage bacteria. Since a time required for centrifugation depends on a degree of clogging of the filter by components of the test sample, it is necessary to grasp a time for filtering the entire amount of reagent B, in advance for each test target. The condition in the present embodiment is 1000 ×g for 1 minute.

After the centrifugation is stopped, the operator takes out from the centrifuge 76 the filtration cartridge 1 to which the first centrifugal filtration attachment 200 is connected. After spraying the filtration cartridge with alcohol to clean the filtration cartridge, the operator introduces the filtration cartridge into the safety cabinet 71. Next, the operator opens the lid 41 of the second filter cup attachment 4 to confirm that there is no residual liquid. Here, when the liquid remains, the centrifugal filtration by the centrifuge 76 may be performed again.

### (Step S24: ATP extraction and ATP Luminescence Measurement)

In step S24, the operator extracts ATP by using the reagent C of the Lumione test kit to complete the preparation of the ATP measurement sample. Details of this step S24 are as follows.

After opening the lid 41 of the second filter cup attachment 4 and confirming that there is no residual liquid, the operator opens the second centrifugal filtration attachment 300. Next, the operator removes the first centrifugal filtration attachment 200 and then attaches the second centrifugal filtration attachment 300 to the filtration cartridge 1. The operator opens the lid 41 and adds the reagent C in an addition amount of 50 µL defined in the manual of the kit by a pipetting operation. Then, the operator closes the lid 41 to the fully closed position to hold the reagent C at a normal temperature for several to 10 minutes in a state of being immersed in the filter 21.

After the reaction with the reagent C, the operator takes out from the safety cabinet 71 the filtration cartridge 1 to which the second centrifugal filtration attachment 300 is connected. Then, the operator sets the filtration cartridge in the centrifuge 76 (zone of grade B), to perform centrifugal filtration. A centrifugal force (×g) can be equal to or less than 2000 ×g not to damage bacteria. Since a time required for centrifugation depends on a degree of clogging of the filter by components of the test sample, it is necessary to grasp a time required for filtering the entire amount (50 µL) of reagent C such that 40 µL or more of reagent C can be collected by centrifugation, in advance for each test target. The condition in the present embodiment is 1000 ×g for 1 minute.

After the centrifugation is stopped, the operator takes out from the centrifuge 76 the filtration cartridge 1 to which the second centrifugal filtration attachment 300 is connected. After spraying the filtration cartridge evenly with alcohol to clean the filtration cartridge, the operator introduces the filtration cartridge into the safety cabinet 71. Next, the operator opens the lid 41 of the second filter cup attachment 4 and confirms that there is no residual liquid. Here, when the liquid remains, the centrifugal filtration by the centrifuge 76 may be performed again. After confirming that there is no residual liquid in the filter 21, the operator closes the lid 41 to the fully closed position and takes out the measurement tube 302 containing the reagent C reaction liquid from the second centrifugal filtration attachment 300. Then, the operator inputs the measurement tube in the dedicated tube rack to cover the tube rack.

Finally, the operator introduces the tube rack into the measurement device 77 installed in grade B to perform ATP measurement.

### <Rapid Microbial Test using Filtration Cartridge, Suction Filtration Attachment, and Centrifugal Filtration Attachment>

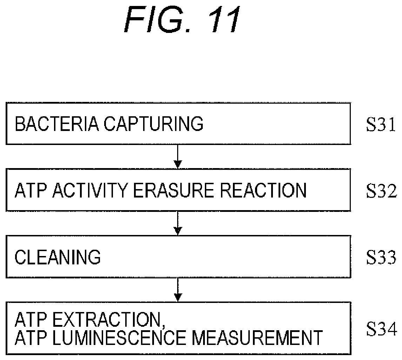

FIG. 11 is a flowchart illustrating the rapid microbial test method using the filtration cartridge 1, the suction filtration attachment 100 (FIG. 5), the first centrifugal filtration attachment 200 (FIG. 6A) and the second centrifugal filtration attachment 300 (FIG. 6B).

This method is also a test method using a funnel, and is suitable for filtering a large volume of test sample. In the reagent reaction using the reagent A, the reagent B, and the reagent C, centrifugal filtration has better liquid breakage and less carryover than suction filtration. Thus, there is an advantage that higher accurate and higher sensitive test can be performed in many cases. On the other hand, since centrifugal filtration applies a load to the filter 21 rather than suction filtration, it is necessary to consider a case where viable bacteria are damaged or bacteria are dead. Thus, it is necessary to determine centrifugation conditions in consideration of a centrifugal force (× g), a centrifugation time, a type of the test sample, and damage to a body cell to be detected.

### (Step S31: Bacteria Capturing)

In step S31, the operator captures bacteria on the filter 21 of the filtration cartridge 1. Details of this step S31 are as follows.

First, the operator introduces into the safety cabinet 71 the test sample, the filtration cartridge 1, the suction filtration attachment 100, the first centrifugal filtration attachment 200, the second centrifugal filtration attachment 300, the funnel, the test reagents, and the sample and reagent dispensing tool. Then, the operator opens the microbial test filtration cartridge 1, the suction filtration attachment 100, and the funnel. Further, the operator removes the sealing material 35 of the filtration nozzle 31 to connect the filtration cartridge 1 and the suction filtration attachment 100. The operator opens the lid 41 of the second filter cup attachment 4 to insert the funnel into the filter cup 2.

Next, as preparation for the filtration step of the test sample, the operator pulls out only the pipe connector 103 attached to the suction filtration attachment 100 from the front shutter of the safety cabinet 71 to the zone of grade B. Then, the operator connects the pipe connector to the suction pump tube 74 in the zone of grade B to drive the suction pump. Next, in the safety cabinet 71, the operator opens a cap of the funnel and injects the test sample into the funnel by a pipetting operation from the container containing the test sample or directly pours the test sample into the funnel from the container containing the test sample. When the test sample is directly poured, it is convenient to pour a required amount of test sample based on a scale of the funnel. After injecting a required amount of test sample into the funnel, the operator closes the lid to the semi-closed position in order to prevent contamination while creating a differential pressure with the cap of the funnel. Then, the operator opens the opening and closing valve 102 of the suction filtration attachment 100 to start the filtration of the test sample. After starting the filtration, the operator visually observes that the sample in the transparent or translucent container of the funnel decreases and visually observes the flow of the liquid in the suction pump tube 74.

When it is determined that the entire amount of test sample is filtered, the operator removes the funnel and opens the lid 41 of the second filter cup attachment 4, to confirm that the liquid does not remain on the filter 21. Then, the operator closes the lid 41 to the fully closed position. Since there is no remaining amount, the operator determines that the filtration is completed. Then, the operator closes the opening and closing valve 102 of the suction filtration attachment 100 and disconnects the suction filtration attachment 100 and the suction pump 72 to stop the filtration.

The operation using the suction filtration attachment 100 has been described so far. In the operation, the liquid component of the test sample (for example, 100 mL or more of injection water) passes through the filter 21. When there are bacteria in the filter 21, the bacteria are concentrated and captured by the filter 21. When the reaction of the reagent A in next step S32 is performed, in order to avoid dilution of a reagent concentration caused by carryover by a minute amount of test sample as much as possible, the reagent A may be added after the residual liquid amount of the test sample is reduced as much as possible, by replacing the suction filtration attachment 100 with the first centrifugal filtration attachment 200, and by adding the filtration step using the centrifugal force.

### (Step S32: ATP Activity Erasure Reaction)

In step S32, the operator performs the ATP activity erasure reaction by using the reagent A of the Lumione test kit. Details of this step S32 are as follows.

The operator opens the first centrifugal filtration attachment 200, removes the suction filtration attachment 100, and attaches the first centrifugal filtration attachment 200 to the filtration cartridge 1.

Subsequent operations are similar to the operations in step S22 illustrated in FIG. 10. Thus, description thereof is omitted.

### (Step S33: Cleaning)

Step S33 is similar to step S23 illustrated in FIG. 10. Thus, description thereof is omitted.

### (Step S34: ATP Extraction and ATP Luminescence Measurement)

Step S34 is similar to step S24 illustrated in FIG. 10. Thus, description thereof is omitted.

### <Conclusion of Fourth Embodiment>

As described above, the filtration cartridge 1 can easily open and close the lid 41. The operation of dispensing the test sample and the reagents is performed in the zone of grade A. In performing the operation using the device such as the centrifuge 76 or the incubator 75 used for a pre-treatment of a specimen, it is possible to bring the device in a direct support zone of grade B in grade A in a sealed state where the lid 41 is closed, or another support zone (grade C or D) in some cases. Accordingly, it is not necessary to bring the device such as the centrifuge 76 or the incubator 75 into the grade A. It is also possible to easily perform the sterile operation without increasing a risk of environmental break due to degradation of a completely sterile environment in grade A due to bringing the device.

In the fourth embodiment, although the microbial test method by the ATP luminescence measurement has been described, the microbial test method using the filtration cartridge 1 can also be applied to other rapid microbial test methods (solid phase cytometry, flow cytometry, immunological methods, nucleic acid amplification methods, fluorescence (staining) methods, and the like).

### [Fifth Embodiment]

In a fifth embodiment, an automated device that automatically performs the microbial test method in FIG. 8 is proposed.

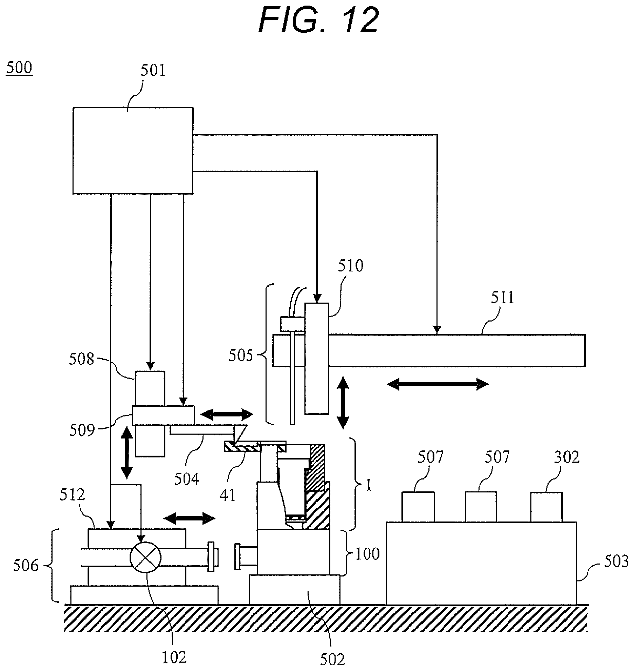

FIG. 12 is a schematic diagram illustrating a microbial test apparatus 500 according to a fifth embodiment. As illustrated in FIG. 12, the microbial test apparatus 500 includes a control device 501, a stage 502, a tube rack 503, a lid opening and closing lever 504, a dispenser 505, and a movable valve waste liquid port 506.

The filtration cartridge 1 to which the suction filtration attachment 100 is connected is placed on the stage 502.

A reagent tube 507 in which the reagent is accommodated and the measurement tube 302 in which the test sample is accommodated are arranged in the tube rack 503.

The lid opening and closing lever 504 is configured to be movable in three axial directions by actuators 508 and 509, and is used to open and close the lid 41 of the filtration cartridge 1.

The dispenser 505 is configured to be movable in three axial directions by actuators 510 and 511. The dispenser 505 dispenses the test sample from the measurement tube 302 to the filtration cartridge 1, and dispenses the reagent from the reagent tube 507 to the filtration cartridge 1.

The movable valve waste liquid port 506 is provide with the opening and closing valve 102 of the suction filtration attachment 100. The movable valve waste liquid port 506 is configured to be movable in a horizontal direction by an actuator 512. The actuator 512 also controls the opening and closing of the opening and closing valve 102.

The control device 501 controls operations of the actuators 508 to 512 based on a predetermined instruction value.

The microbial test apparatus 500 can be installed, for example, in the above-described zone of grade A. The microbial test apparatus 500 can be defined as an apparatus that performs only operations to be performed in the zone of grade A (for example, the operation of dispensing the test sample and the reagent), but may be defined as an apparatus that can perform operations that can be performed in the zone of grade B.

### <Microbial Test Method>

The microbial test method according to the present embodiment is substantially similar to the method illustrated in FIG. 8. The control device 501 operates the actuators 508 to 512 to perform the operations by the operator.

### <Conclusion of Fifth Embodiment>

As described above, the microbial test apparatus 500 according to the fifth embodiment automatically performs the microbial test using the filtration cartridge 1 and the suction filtration attachment 100. As a result, since the number of manual operations by the operator is reduced, the microbial test is more convenient.

### [Sixth Embodiment]

In a sixth embodiment, an automated device that automatically performs the microbial test method in FIG. 10 is proposed.

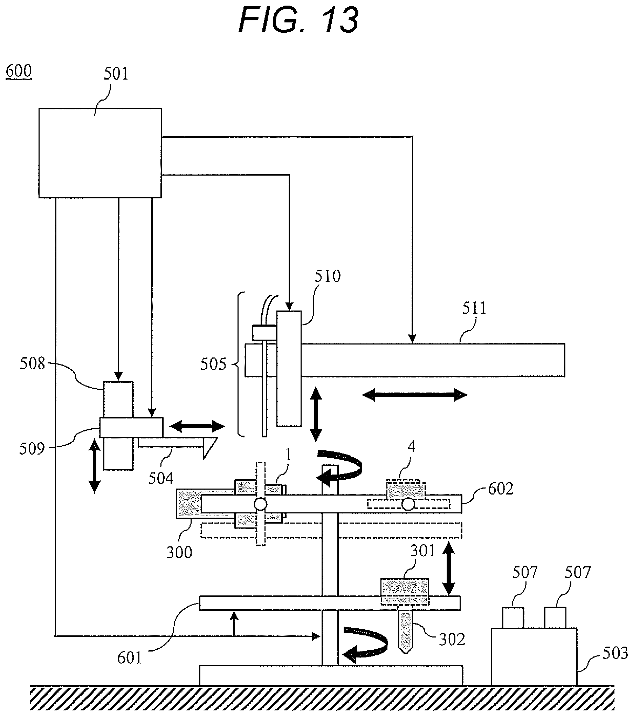

FIG. 13 is a schematic diagram illustrating a microbial test apparatus 600 according to a sixth embodiment. As illustrated in FIG. 13, in the microbial test apparatus 600, a control device 501, a tube rack 503, a lid opening and closing lever 504, a dispenser 505, and actuators 508 to 511 are similar to the components of the microbial test apparatus 500 (FIG. 12) according to the fifth embodiment.

The microbial test apparatus 600 includes a centrifugal filtration attachment rack 601 and a swing rotor 602. The centrifugal filtration attachment rack 601 holds the first centrifugal filtration attachment 200 (not illustrated) and the second centrifugal filtration attachment 300. The swing rotor 602 holds the filtration cartridge 1. The centrifugal filtration attachment rack 601 and the swing rotor 602 are configured to be rotatable. The centrifugal filtration attachment rack 601 is configured to be vertically movable. The swing rotor 602 is configured to change an orientation of the filtration cartridge 1. The driving of the centrifugal filtration attachment rack 601 and the swing rotor 602 is controlled by the control device 501.

When the filtration cartridge 1 is attached to the first centrifugal filtration attachment 200 and the second centrifugal filtration attachment 300, the centrifugal filtration attachment rack 601 is raised in a state where the second filter cup attachment 4 is aligned with the first fitting portion 201 and the second fitting portion 301.

When a centrifugal operation of the filtration cartridge 1 is required, the lid of the filtration cartridge 1 to which the second centrifugal filtration attachment 300 is attached is set to the fully closed position. Then, an orientation of the filtration cartridge 1 is changed in the horizontal direction and the filtration cartridge 1 is rotated by the swing rotor 602. As a result, since a centrifugal force is generated in a radial direction of the swing rotor 602, the liquid added to the filtration cartridge 1 is filtered. As described above, when the centrifugal filtration is performed, since it is not necessary to move the filtration cartridge 1 to the centrifuge, there is no risk of contamination associated with the movement, and the operation is also simplified.

### <Microbial Test Method>

The microbial test method according to the present embodiment is substantially similar to the method illustrated in FIG. 10. The operations by the operator are executed by the control device 501 operating the actuators 508 to 511, the centrifugal filtration attachment rack 601, and the swing rotor 602.

### <Conclusion of Sixth Embodiment>

As described above, the microbial test apparatus 600 according to the sixth embodiment automatically performs the microbial test using the filtration cartridge 1 and the second centrifugal filtration attachment 300. As a result, since the number of manual operations by the operator is reduced, the microbial test becomes more simplified.

### <Modification Examples of Fifth and Sixth Embodiments>

Although not illustrated, the microbial test method (method using both the suction filtration attachment 100 and the centrifugal filtration attachments 200 and 300) illustrated in FIG. 11 can also be performed by using the microbial test apparatus including both the configuration of the fifth embodiment and the configuration of the sixth embodiment.

### [Modification Example]

The present disclosure is not limited to the above-described embodiments, and includes various modification examples. The aforementioned embodiments are described in detail in order to facilitate easy understanding of the present disclosure, and are not limited to necessarily include all the described components. A part of a certain embodiment can be replaced with a configuration of another embodiment. The configuration of another embodiment can be added to the configuration of a certain embodiment. A part of the configuration of another embodiment can be added to, deleted from, or replaced with a part of the configuration of each embodiment.

### Reference Signs List

- 1: filtration cartridge
- 2: filter cup
- 21: filter
- 22: flange
- 23: container portion
- 24: support plate
- 3: first filter cup attachment
- 31: filtration nozzle
- 32: body portion
- 33: attachment joint hole
- 34: filter cup insertion portion
- 35: sealing material
- 36: recessed portion
- 37: protrusion portion
- 4: second filter cup attachment
- 41: lid
- 42: body portion
- 43: filter cup introduction hole
- 44: flange holding portion
- 45: first fixing member
- 46: lid fixing hole
- 47: second fixing member
- 48: knob
- 49: protrusion portion
- 50: claw-like connector
- 51: tubular connector
- 52: hinge mechanism
- 53: knob
- 70: biological clean room
- 71: safety cabinet
- 72: suction pump
- 73: waste liquid trap
- 74: suction pump tube
- 75: incubator
- 76: centrifuge
- 77: measurement device
- 78: pass box
- 79: sterile operation zone
- 100: suction filtration attachment
- 101: support block
- 102: opening and closing valve
- 103: pipe connector
- 104: flow path
- 200: first centrifugal filtration attachment
- 201: first fitting portion
- 202: waste liquid tube
- 300: second centrifugal filtration attachment
- 301: second fitting portion
- 302: measurement tube
- 500: microbial test apparatus
- 600: microbial test apparatus

## Claims

1. A filtration cartridge, comprising:
a filter cup with a filter capturing microorganisms;
a first filter cup attachment that covers a lower portion of the filter cup and has at least one through-hole in a bottom surface portion; and
a second filter cup attachment, being connected to the first filter cup attachment, that covers an upper portion of the filter cup, and that has a lid installed at a position away from an opening surface of the filter cup.

2. The filtration cartridge according to claim 1, wherein the through-hole is able to be connected to a suction filtration attachment, and wherein the suction filtration attachment has a connection structure portion connectable to a suction device.

3. The filtration cartridge according to claim 2, wherein the suction filtration attachment further has a flow path that allows the through-hole and the connection structure portion to communicate with each other, and wherein the connection structure portion has a valve that controls opening and blocking of the flow path.

4. The filtration cartridge according to claim 1, wherein the through-hole is connectable to a centrifugal filtration attachment.

5. The filtration cartridge according to claim 4, wherein the centrifugal filtration attachment has a container communicating with the through-hole.

6. The filtration cartridge according to claim 1, wherein a closed state of the lid of the second filter cup attachment includes a first closed state in which a gap is formed and a second closed state as a sealed state.

7. The filtration cartridge according to claim 1, wherein the lid moves in parallel with the opening surface of the filter cup.

8. The filtration cartridge according to claim 1, wherein the lid is opened and closed by a hinge mechanism.

9. The filtration cartridge according to claim 1, wherein the through-hole is a filtration nozzle through which a liquid is able to pass from an upper portion to a lower portion of the filter.

10. The filtration cartridge according to claim 1, wherein an opened portion of the through-hole is sealed with a sealing material.

11. The filtration cartridge according to claim 1, wherein
the filter cup includes a support plate that fixes the filter, and
the support plate has a through-hole through which the liquid having passed through the filter passes.

12. The filtration cartridge according to claim 1, wherein the filtration cartridge is disposable.

13. The filtration cartridge according to claim 1, wherein the filtration cartridge is at least doubly packaged.

14. A microbial test method, comprising:
connecting the filtration cartridge according to claim 1 and a suction filtration attachment in a sterile operation zone;
connecting the suction filtration attachment to a suction device;
opening the lid of the second filter cup attachment to add a sample to the filter cup; and
closing the lid and driving the suction device to filter the sample so that microorganisms are captured by the filter.

15. The microbial test method according to claim 14, further comprising:
opening the lid in the sterile operation zone after the capturing of the microorganisms, adding a first reagent to the filter cup, closing the lid, and then performing reaction;
filtering, after the reaction, the first reagent by driving the suction device;
opening the lid in the sterile operation zone to add a second reagent to the filter cup;
closing the lid to perform reaction; and
opening the lid in the sterile operation zone to distribute a reaction liquid of the second reagent and the microorganisms from above the filter of the filter cup.

16. A microbial test method, comprising:
connecting the filtration cartridge according to claim 1 and a first centrifugal filtration attachment in a sterile operation zone;
opening the lid of the second filter cup attachment to add a sample to the filter cup; and
closing the lid to set the filtration cartridge to which the first centrifugal filtration attachment is connected in a centrifuge installed in a peripheral zone of the sterile operation zone;
performing centrifugal filtration of the sample so that microorganisms are captured by the filter.

17. The microbial test method according to claim 16, further comprising:
opening the lid in the sterile operation zone after the capturing of the microorganisms, to add a first reagent to the filter cup;
closing the lid to perform reaction;
setting the filtration cartridge in the centrifuge after the reaction;
performing centrifugal filtration of a first reaction liquid of the first reagent and the microorganisms;
removing the first centrifugal filtration attachment in the sterile operation zone;
connecting the second centrifugal filtration attachment to the filtration cartridge;
opening the lid to add a second sample to the filter cup, closing the lid;
performing reaction;
setting the filtration cartridge to which the second centrifugal filtration attachment is connected in the centrifuge after the reaction to filter a second reaction liquid of the second reagent and the microorganisms by centrifugal filtration; and,
collecting filtrate in a container provided in the second centrifugal filtration attachment.
